# EUROPEAN PATENT APPLICATION

(11) **EP 1 672 081 A1**
(43) Date of publication of application: **21.06.2006**
(21) Application number: 04792227.3
(22) Date of filing: 08.10.2004
(51) Int. Cl.: C12Q 1/68

(54) **PRIMER FOR DETECTING ALICYCLOBACILLUS**

(30) Priority: 08.10.2003 JP 2003349772
(71) Applicant: Sapporo Breweries Limited, Tokyo 150-8522 (JP); Eiken Chemical Co., Ltd., Bunkyo-ku, Tokyo 1138408 (JP)
(72) Inventor: Nakakita, Yasukazu, Sapporo Breweries Limited, Yaizu-shi, Shizuoka 4250013 (JP); Ogawa, Masahiro, Sapporo Breweries Limited, Yaizu-shi, Shizuoka 4250013 (JP); Tsuchiya, Youichi, Sapporo Breweries Limited, Yaizu-shi, Shizuoka 4250013 (JP)
(74) Representative: Vossius & Partner
(86) International application number: PCT/JP2004/014959
(87) International publication number: WO 2005/035789

(57) **Abstract**

Primers specific for an *Alicyclobacillus* spp. are used to amplify specific regions of 16S rDNA gene of the *Alicyclobacillus* spp., and the presence or absence of the amplified product is confirmed to detect the *Alicyclobacillus* spp. (especially *Alicyclobacillus acidoterrestris).*

## Description

### Technical Field

The present invention relates to primers specific for 16S rDNA of *Alicyclobacillus* spp. or *Alicyclobacillus acidoterrestris.* More specifically, the invention relates to primers or primer sets for detection of *Alicyclobacillus* spp. which is capable of amplifying specific regions of 16S rDNA of *Alicyclobacillus* bacteria, and to primer sets for detection of *Alicyclobacillus acidoterrestris* which is capable of amplifying specific regions of 16S rDNA of *Alicyclobacillus acidoterrestris.* The invention still further relates to a method for detection of *Alicyclobacillus* spp. and *Alicyclobacillus acidoterrestris* and identifying of species, wherein the aforementioned primers or primer sets for detection of the bacteria is used in Loop-Mediated Isothermal Amplification method (hereinafter abbreviated as LAMP method).

### Background Art

Bacteria of the *Alicyclobacillus* spp. are aerobic thermoacidophilic spore-forming bacteria isolated from soil, hot springs and elsewhere. It is known that soil-inhabiting *Alicyclobacillus* spp. which adhere to harvested fruit and contaminate juice from squeezing processes are able to survive as heat-resistant bacterial spores during production of beverages and foods from such juice, even when heat sterilization is carried out during production, and can germinate and proliferate in the product to cause spoilage. In particular, *Alicyclobacillus acidoterrestris* is a bacterium of the *Alicyclobacillus* spp. which causes an offensive odor named guaiacol odor and has been noted as a problem based on reports implicating it as a causative bacterium of food contamination.

Strategies for avoiding food contamination include microorganic test for detection and identification of *Alicyclobacillus* spp., and several methods for this have been proposed. BAM medium (Non-patent document 1) and Semi-synthetic medium (Non-patent document 2), for example, have been reported as detection media, but because the compositions of these media are complex and several days are necessary to obtain examination results, they have not been well suited for routine microorganic test.

On the other hand, since physiological and biochemical properties can differ minimally between species while variation of the properties is also found within strains, methods for identifying bacterial species cannot easily discern between different species based solely on such properties (Non-patent document 3).

Identification methods using genetic analysis techniques have also been proposed. For example, methods using PCR method (Patent document 1, Non-patent document 4), methods using RAPD method (Non-patent document 5) and methods using 16S rDNA sequence comparison (Non-patent document 6) exist, but all such methods require expensive equipment and entail complicated procedures, and are therefore problematic when used for routine microorganic test.

Patent document 1: WO 01/68914
Non-patent document 1: G. Deinhard et al., System. Appl. Microbiol., 10, 47-53, 1987
Non-patent document 2: B. Hippchen et al., Arch. Microbiol., 129, 53-55, 1981
Non-patent document 3: K. Goto, J. Antibact. Antifung. Agents., 28(8), 499-508, 2000
Non-patent document 4: K. Yamazaki et al., Lett. Appl. Microbiol., 23, 350-354, 1996
Non-patent document 5: K. Yamazaki et al., Biosci. Biotech. Biochem., 61(6), 1016-1018, 1997
Non-patent document 6: K. Goto et al., J. Gen. Appl. Microbiol., 48, 243-250, 2002

### Disclosure of the Invention

### Problems to be Solved by the Invention

In light of the problems described above, it has been desired to develop a detection method and identification method for *Alicyclobacillus* (especially *Alicyclobacillus acidoterrestris)* bacteria in a convenient and rapid manner, by a different approach than the methods of the prior art. It is therefore an object of the invention to solve the aforementioned problems by providing a convenient, rapid method of detecting and identifying of presence or absence of *Alicyclobacillus* spp. in food specimens such as beverages and fruit juices.

### Means for Solving the Problems

As a result of much avid research on the problems described above, the present inventors have discovered that detection and identification of *Alicyclobacillus* spp. and *Alicyclobacillus acidoterrestris* can be accomplished in a convenient and rapid manner by utilizing Loop-Mediated Isothermal Amplification (LAMP) method (WO00/28082), which is an isothermal gene amplification reaction.

In other words, the invention provides primers specific for 16S rDNA *of Alicyclobacillus* spp. or *Alicyclobacillus acidoterrestris.*

According to the invention, there is provided a detection and identification method for *Alicyclobacillus* spp. (preferably *Alicyclobacillus acidoterrestris)* specimens, characterized by using a specific region of the bacterial 16S rDNA as a target, selectively amplifying the specific region of the 16S rDNA by the LAMP method using primers specific for the 16S rDNA, and confirming the presence or absence of the amplified product.

More specifically, the invention provides primers or primer sets for detection of *Alicyclobacillus* spp. or *Alicyclobacillus acidoterrestris,* and a detection and identification method for *Alicyclobacillus* spp. or *Alicyclobacillus acidoterrestris* which employs them, according to the following (1) to (13).

(1) A primer which amplifies the base sequence of a target region selected from any portion of the nucleic acid sequence from base 81 to base 934 of the 16S rDNA of an *Alicyclobacillus* spp., or its complementary strand, the primers being characterized by comprising:
(a) a base sequence which functions as a primer by annealing to 16S rDNA of the *Alicyclobacillus* spp., as the first segment, and
(b) a base sequence located at the 5' end of the first segment which is complementary to the nucleic acid sequence at the 3' end of the first segment, as the second segment.
   (2) A primer set for detection of an *Alicyclobacillus* spp., which comprises an oligonucleotide set consisting of the nucleic acid sequences set forth in SEQ ID NOS: 1 to 4 and is capable of amplifying a specific region of 16S rDNA of the *Alicyclobacillus* spp..
   (3) A primer set for detection *of Alicyclobacillus acidoterrestris,* which comprises an oligonucleotide set consisting of the nucleic acid sequences set forth in SEQ ID NOS: 5 to 8 and is capable of amplifying a specific region of 16S rDNA of *Alicyclobacillus acidoterrestris.*
   (4) A primer set for detection of *Alicyclobacillus acidoterrestris,* which comprises an oligonucleotide set consisting of the nucleic acid sequences set forth in SEQ ID NOS: 9 to 13 and is capable of amplifying a specific region of 16S rDNA of *Alicyclobacillus acidoterrestris.*
   (5) A method for detection of *an Alicyclobacillus* spp. present in a specimen, characterized by using a specific region of 16S rDNA of the *Alicyclobacillus* spp. as a target, selectively amplifying the specific region of 16S rDNA by the LAMP method using a primer according to (1) above, and confirming the presence or absence of the amplified product.
   (6) A method for detection of an *Alicyclobacillus* spp. present in a specimen, characterized by using a specific region of 16S rDNA of the *Alicyclobacillus* spp. as a target, selectively amplifying the specific region of 16S rDNA by the LAMP method using a primer set according to (2) above, and confirming the presence or absence of the amplified product.
   (7) A method for detection of *Alicyclobacillus acidoterrestris* present in a specimen, characterized by using a specific region of 16S rDNA of *Alicyclobacillus acidoterrestris* as a target, selectively amplifying the specific region of 16S rDNA by the LAMP method using a primer set according to (3) above, and confirming the presence or absence of the amplified product.
   (8) A method for detection of *Alicyclobacillus acidoterrestris* present in a specimen, characterized by using a specific region of 16S rDNA of *Alicyclobacillus acidoterrestris* as a target, selectively amplifying the specific region of 16S rDNA by the LAMP method using a primer set according to (4) above, and confirming the presence or absence of the amplified product.
   (9) A method for identification of an *Alicyclobacillus* spp., characterized by carrying out enrichment cultivation of a specimen, separating a DNA sample from the appearing bacteria, subjecting the DNA sample to amplification reaction by the LAMP method using a primer according to (1) above, amplifying the specific region of 16S rDNA of the *Alicyclobacillus* spp., and confirming the presence or absence of the amplified product.
   (10) A method for identification of an *Alicyclobacillus* spp., characterized by carrying out enrichment cultivation of a specimen, separating a DNA sample from the appearing bacteria, subjecting the DNA sample to amplification reaction by the LAMP method using a primer set according to (2) above, amplifying the specific region of 16S rDNA of the *Alicyclobacillus* spp., and confirming the presence or absence of the amplified product.
   (11) A method for identification of *Alicyclobacillus acidoterrestris,* characterized by carrying out enrichment cultivation of a specimen, separating a DNA sample from the appearing bacteria, subjecting the DNA sample to amplification reaction by the LAMP method using a primer set according to (3) above, amplifying the specific region of 16S rDNA of *Alicyclobacillus acidoterrestris,* and confirming the presence or absence of the amplified product.
   (12) A method for identification of *Alicyclobacillus acidoterrestris,* characterized by carrying out enrichment cultivation of a specimen, separating a DNA sample from the appearing bacteria, subjecting the DNA sample to amplification reaction by the LAMP method using a primer set according to (4) above, amplifying the specific region of 16S rDNA of *Alicyclobacillus acidoterrestris,* and confirming the presence or absence of the amplified product.
   (13) A kit for detection of an *Alicyclobacillus* spp. or an *Alicyclobacillus acidoterrestris,* characterized by comprising at least a primer or a primer set according to any one of(1) to (4) above, strand-displacing DNA polymerase, dNTPs and reaction buffer.

When primers or primer sets prescribed by (1) to (4) above is used in the LAMP method, the primers or the primers set anneal to specific regions of 16S rDNA of the *Alicyclobacillus* spp.. Amplification under the amplification conditions established for the LAMP method results in amplification of the specific gene region. Presence or absence of the amplified product is confirmed by electrophoresis or simple testing. It is thus possible to detect an *Alicyclobacillus* spp.. Since the two primer sets of (3) or (4) above are specific for *Alicyclobacillus acidoterrestris* (16S rDNA), they specifically detect only *Alicyclobacillus acidoterrestris* among *Alicyclobacillus* spp., and therefore these primer sets are able to distinguish between *Alicyclobacillus acidoterrestris* and other *Alicyclobacillus* spp..

Moreover, the detection method of the invention may be applied to particular specimens (for example, beverage samples), by culturing contaminating bacteria taken from the specimen, separating a DNA sample therefrom, reacting the primers or the primer sets prescribed by (1) to (4) above with the DNA sample and confirming the presence or absence of DNA amplified product amplified in the same manner. It is thus possible to detect *Alicyclobacillus* spp. or *Alicyclobacillus acidoterrestris.*

### Effect of the Invention

The present invention allows detection of an *Alicyclobacillus* spp. by using primer sets consisting of sets of oligonucleotides comprising the nucleic acid sequences set forth in SEQ ID NOS: 1-4, 5-8 or 9-13 in the LAMP method for amplification of specific regions of 16S rDNA of an *Alicyclobacillus* spp. (preferably *Alicyclobacillus acidoterrestris).*

The method for detection of an *Alicyclobacillus* spp. according to the invention employs the LAMP method on a DNA sample obtained from a specimen, using primer sets consisting of sets of oligonucleotides comprising the nucleic acid sequences set forth in SEQ ID NOS: 1-4, 5-8 or 9-13, and confirms the presence or absence of amplified product, whereby it is possible to achieve easy, rapid and reliable detection and identification of *Alicyclobacillus* spp. (particularly harmful *Alicyclobacillus acidoterrestris).*

### Brief Description of the Drawings

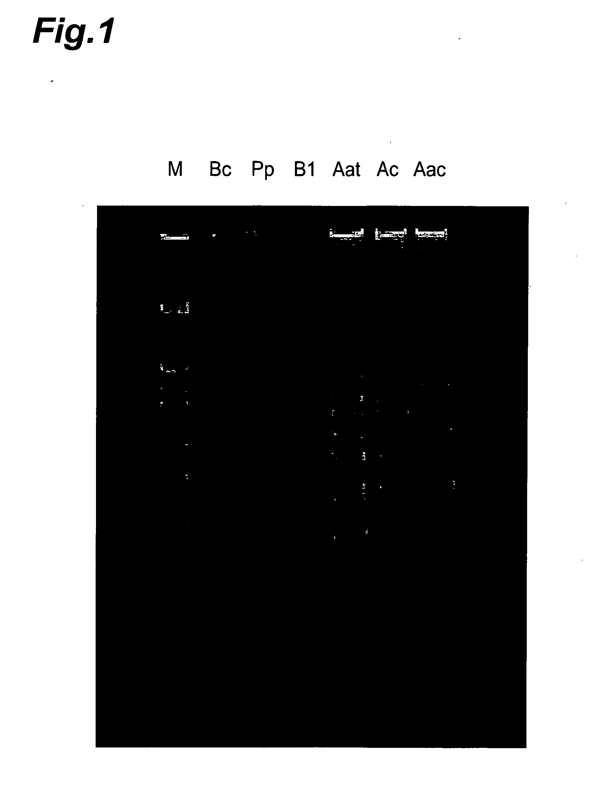

Fig. 1 is an electrophoresis diagram showing the results of isothermal gene amplification with Al primer set. M: Marker, Bc: *Bacillus coagulans* ID237, Pp: *Paenibacillus polymyxa* ID322-1, B1: *Bacillus licheniformis* ID354-1, Aat: *Alicyclobacillus acidoterrestris* DSM2498, Ac: *Alicyclobacillus cycloheptanicus* NBRC15310, Aac: *Alicyclobacillus acidocaldarius* JCM5260.

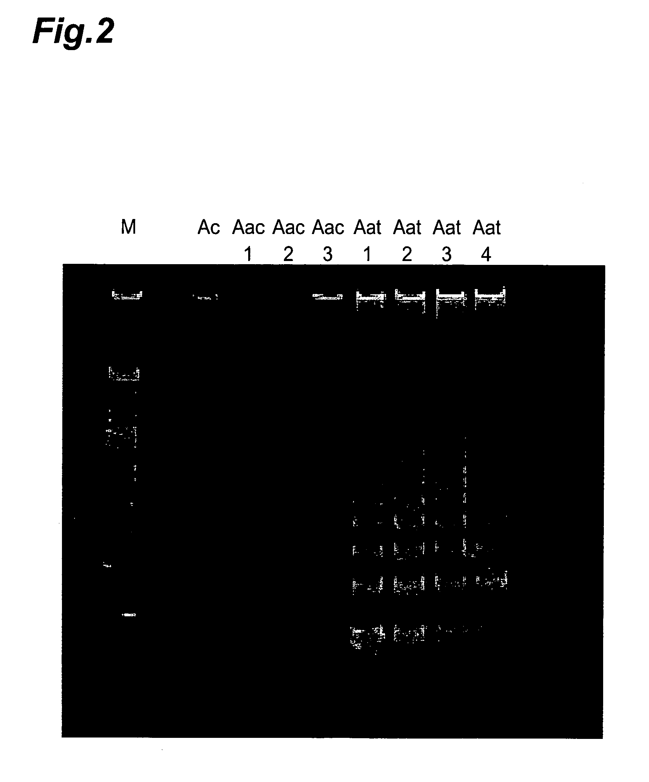

Fig. 2 is an electrophoresis diagram showing the results of isothermal gene amplification with Alat primer set. Lane M: Marker, Ac: *Alicyclobacillus cycloheptanicus* NBRC15310, Aac1: *Alicyclobacillus acidocaldarius* JCM5260, Aac2: *Alicyclobacillus acidocaldarius* ID313-1, Aac3: *Alicyclobacillus acidocaldarius* ID313-3, Aat1: *Alicyclobacillus cycloheptanicus* NBRC15310, Aat2: *Alicyclobacillus acidocaldarius* JCM5260, Aat3: *Alicyclobacillus acidoterrestris* ID313-4, Aat4: *Alicyclobacillus acidoterrestris* ID331-1.

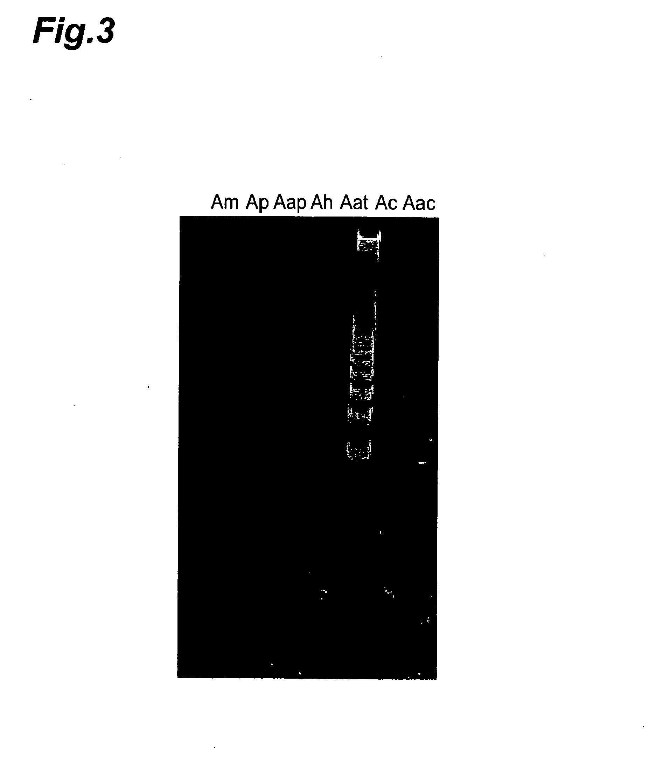

Fig. 3 is an electrophoresis diagram showing the results of isothermal gene amplification with rAlat primer set. Am: *Alicyclobacillus mali* IAM14936, Ap: *Alicyclobacillus pomorum* IAM14988, Aap: *Alicyclobacillus acidiphilus* IAM14883, Aat: *Alicyclobacillus acidoterrestris* DSM2498, Ac: *Alicyclobacillus cycloheptanicus* NBRC 15310, Aac: *Alicyclobacillus acidocaldarius* JCM5260, Aac: *Alicyclobacillus acidocaldarius* JCM5260.

### Best Mode for Carrying Out the Invention

In order to discern whether or not *Alicyclobacillus* spp., and especially *Alicyclobacillus acidoterrestris,* are present in food products including fruit juices and fruit beverages (also collectively referred to as beverages), the present invention is on the basis of carrying out amplification of a specific region of 16S rDNA of the bacteria by isothermal gene amplification by the LAMP method, using oligonucleotide primers specific for the bacteria, and confirming the presence or absence of amplified product.

In beverage factories, the presence of *Alicyclobacillus* spp. is checked upon reception of fruit juice and during microbial examination of fruit beverages. Generally, the beverage specimen is filtered with a membrane filter, and the bacteria-trapped membrane filter is placed in *Alicyclobacillus-culturing* YSG agar medium (yeast extract: 0.2%, glucose: 0.1%, soluble starch: 0.2%, agar: 1.5%, pH 4.0) and cultured at 45°C for 3-5 days, according to "Thermo Acidophilic Bacilli Standardized Examination Method" (Kaju Kyokaiho, Mar. No. 535, 4-12, 2003). The appearing bacteria are discerned as being *Alicyclobacillus acidoterrestris* or not by a temperature gradient method (which requires 18-20 hours for discernment) or the peroxidase method (which requires 1-3 hours for discernment).

In the case of a specimen that is not suitable for membrane filtration, the specimen is added to YSG liquid medium and subjected to enrichment culture at 45°C for 3-5 days, after which the culture solution is coated and spread onto YSG agar medium and culturing is continued at 45°C for 3-5 days. The appearing bacteria are discerned as being *Alicyclobacillus acidoterrestris* or not by a temperature gradient method or peroxidase method, in the same manner.

The bacteria provided as a sample for the detection and identification method of the invention may be bacteria appearing in YSG agar medium, or bacteria present in enrichment culture solution.

The term "specimen" as used throughout the present specification is a sample for the detection and identification method of the invention which is suspected of including harmful *Alicyclobacillus acidoterrestris,* and it is not particularly restricted. For example, there may be mentioned fruit juices (raw materials) and fruit juice-containing beverages. The term "identification" as used throughout the present specification may specifically refer to distinguishing between and detecting *Alicyclobacillus acidoterrestris* and other *Alicyclobacillus* spp. in a specimen containing one or more species of *Alicyclobacillus* spp., but generally it refers to identification of *Alicyclobacillus* spp. or *Alicyclobacillus acidoterrestris* as targets of detection among multiple bacterial species. The term "discernment" as used throughout the present specification refers to determining whether or not a detected bacterium is an *Alicyclobacillus* spp. or whether it is an *Alicyclobacillus acidoterrestris,* and it will sometimes be used synonymously with "detection".

The LAMP method carried out for the invention differs from PCR in that it does not require temperature regulation (cycling) during the amplification step, and it is a gene amplification method whereby amplification is accomplished at a constant temperature (isothermally) using a single DNA synthase (WO00/28082, mentioned above).

The primers specific to 16S rDNA of an *Alicyclobacillus* spp. according to the invention are designed as a set comprising a total of 4 different primers (FIP, F3, BIP, B3), two different internal primers which form loops and two different external primers, targeted to a specific region. The specific region of the gene to be amplified consists of approximately 100-500 bp. If the nucleic acid sequences of the oligonucleotides of the primers are determined, synthesis of the oligonucleotides themselves can be accomplished using publicly known means, such as an automatic DNA synthesizer by Perkin Elmer.

According to the invention, the primer set specific to an *Alicyclobacillus* spp. may be, for example, the following primer set (Al primer set). Primers specific to an *Alicyclobacillus* spp. are primers capable of specifically amplifying a specific region (141 bp) of 16S rDNA of the bacterium. The total nucleic acid sequence of 16S rDNA is set forth in SEQ ID NO: 14 of the Sequence Listing.

(A1 primer set)
A1-FIP
5'-TTGGGTTTCCTTCGGCACTGAGATACCCTGGTAGTCCACG-3' (SEQ ID NO: 1)
A1-BIP
5'-ATAAGCACTCCGCCTGGGGAGCCCCCGTCAATTCCTTTG-3' (SEQ ID NO: 2)
A1-F3
5'-GTGGGGAGCAAACAGGATT-3' (SEQ ID NO: 3) A1-B3
5'-ACATGCTCCACTGCTTGTG-3' (SEQ ID NO: 4)

According to the invention, the following two primers sets (Alat primer set, rAlat primer set) may be mentioned as examples of primer sets specific only to *Alicyclobacillus acidoterrestris.* Here, primers specific only to *Alicyclobacillus acidoterrestris* are primers capable of specifically amplifying specific regions (139 bp or 146 bp) of 16S rDNA of *Alicyclobacillus acidoterrestris,* without amplifying genes of other *Alicyclobacillus* spp..

(Alat primer set)
Alat-FIP 5'-ACAAGGAGCTTTCCACTCTCCAAGAAGGCCTTCGGGTTGT-3' (SEQ ID NO: 5)
Alat-BIP
5'-TGAGACGGTACCGAGTGAGGACGCTTGCCCCCTACGTATT-3' (SEQ ID NO: 6)
Alat-F3
5'-CAAGCCTGACGGAGCAA-3' (SEQ ID NO: 7)
Alat-B3
5'-CCCAGTGATTCCGGACAA-3' (SEQ ID NO: 8)

(rAlat primer set)
rAlat-FIP
5'-AACACAAGTAGATGCCTACCCGCAATCTGCCTTTCAGACTGG A-3' (SEQ ID NO: 9)
rAlat-BIP
5'-TTGAAAGATGCAACTGCATCGCTCCGTTACCTCACCAACTAG C-3' (SEQ ID NO: 10)
rAlat-F3
5'-CGGACGGGTGAGTAACACG-3' (SEQ ID NO: 11)
rAlat-B 3
5'-TACGCATCGTCGCCTTGG-3' (SEQ ID NO: 12)
rAlat-LoopF
5'-ATTAGCACCCGTTTCCGAGT-3' (SEQ ID NO: 13)

Detection of an *Alicyclobacillus* spp. and/or *Alicyclobacillus acidoterrestris* present in a specimen is carried out using isothermal gene amplification (normally 60-65°C for 15 minutes to 1 hour) by the LAMP method with at least one set of the three primer sets mentioned above. Specifically, a bacterial strain obtained from a specimen or a beverage specimen itself is cultured in an appropriate medium and the cells are collected by centrifugal separation or the like, after which DNA of the bacterial strain is separated by a publicly known method and the DNA is subjected to amplification reaction using the aforementioned primer set. The presence or absence of amplified DNA amplified product may be detected by simple detection based on the LAMP method, or by ordinary electrophoresis. The former simple detection includes 1) visual confirmation based on turbidity of the amplification reaction solution (WO 01/83817), and 2) visual confirmation of a fluorescent intercalator. In either case, the presence or absence of amplified product (the target gene) can be confirmed visually.

### Examples

The present invention will now be explained in greater detail by examples, with the understanding that the invention is in no way limited by the examples.

(Example 1) Amplification of 16S rDNA gene of known strains

### (Primer set)

The *Alicyclobacillus* spp. primer set [Al primer set: AL-FIP (SEQ ID NO: 1), Al-BIP (SEQ ID NO: 2), Al-F3 (SEQ ID NO: 3), Al-B3 (SEQ ID NO: 4)] amplifies a portion of 16S rDNA (141 bp). The *Alicyclobacillus acidoterrestris* primer set 1 [Alat primer set: Alat-FIP (SEQ ID NO: 5), Alat-BIP (SEQ ID NO: 6), Alat-F3 (SEQ ID NO: 7), Alat-B3 (SEQ ID NO: 8)] amplifies a portion of 16S rDNA (139 bp). The *Alicyclobacillus acidoterrestris* primer set 2 [rAlat primer set: rAlat-FIP (SEQ ID NO: 9), rAlat-BIP (SEQ ID NO: 10), rAlat-F3 (SEQ ID NO: 11), rAlat-B3 (SEQ ID NO: 12), rAlat-LoopF (SEQ ID NO: 13)] amplifies a portion of 16S rDNA (146 bp). These primer sets were used for amplification of 16S rDNA of *Alicyclobacillus* spp. and *Alicyclobacillus acidoterrestris,* and for amplification of 16S rDNA of related bacterial species. The strains provided for testing in the examples are listed in Table 1 together with the results for each strain.

### (Preparation of genomic DNA)

The *Alicyclobacillus* spp. were inoculated in YSG agar medium (yeast extract: 0.2%, glucose: 0.1%, soluble starch: 0.2%, agar: 1.5%, pH 4.0) and cultured at 45°C for 2-5 days. The other test strains were inoculated in heart infusion agar medium (product of Eiken Chemical Co., Ltd.) and cultured at 37°C for 2-5 days. Upon completion of culturing, the genomic DNA was extracted from each bacterium using PrepMan™ Ultra (product of Applied Biosystems).

### (Gene amplification by LAMP method)

Gene amplification by LAMP method was carried out using a Loopamp DNA amplification reagent kit (product of Eiken Chemical Co., Ltd.). The aforementioned DNA extraction solution and each primer set were added to the reagent reaction solution and nucleic acid amplification reaction was conducted at 62°C for 60 minutes.

### (Detection of amplified product)

As the amplification reaction proceeds, magnesium pyrophosphate is formed by released pyrophosphoric acid and magnesium ion present in the reaction solution. Thus, the reaction solution becomes turbid only with samples that yield nucleic acid amplification. Observation of turbidity allows discernment of the presence or absence of amplified product. Some of the strains were analyzed by polyacrylamide gel electrophoresis of 1 µl of post-amplification reaction solution. DNA bands were confirmed by staining for 10 minutes with ethidium bromide solution followed by ultraviolet irradiation (results are shown in Figs. 1 to 3).

The results of amplification with the Al primers and Alat primers are summarized in Table 1.

**[Table 1]**

| Test strain | Amplification result | |
|---|---|---|
| | Al primers | Alat primers |
| *Alicyclobacillus cycloheptanicus* IF015310 | + | - |
| *Alicyclobacillus acidocaldarius* JCM5260 | + | - |
| *Alicyclobacillus acidocaldarius* ID313-1 | + | - |
| *Alicyclobacillus acidocaldarius* ID313-3 | + | - |
| *Alicyclobacillus acidoterrestris* DSM2498 | + | + |
| *Alicyclobacillus acidoterrestris* ID313-2 | + | + |
| *Alicyclobacillus acidoterrestris* ID313-4 | + | + |
| *Alicyclobacillus acidoterrestris* ID331-1 | + | + |
| *Bacillus coagulans* AHU1366 | - | |
| *Bacillus coagulans* AHU1367 | - | - |
| *Bacillus coagulans* ID237 | - | - |
| *Bacillus licheniformis* AHU1371 | - | - |
| *Bacillus licheniformis* ID351-4 | - | - |
| *Bacillus polymyxa* IAM 1189 | - | - |
| *Paenibacillus polymyxa* ID322-1 | - | - |

| | | |
|---|---|---|
| +: amplified -: not amplified | | |
| NBRC: Biological Resource Center, Biotechnology Center, National Institute of Technology and Evaluation, Tsukuba, Japan | | |
| JCM: Japan Collection of Microorganisms, Saitama, Japan | | |
| AHU: Faculty of Agriculture, Hokkaido University, Japan | | |
| IAM: IAM Culture Collection, Institute of Molecular and Cellular Biosciences, The University of Tokyo, Japan | | |
| DSM: German Collection of Microorganisms and Cell Cultures, Germany | | |
| ID: Sapporo Beer isolate | | |

As shown in Table 1, amplified product was confirmed for all of the test strains belonging to the *Alicyclobacillus* spp. when using the Al primer set. When using the Alat primer set, however, amplified product was confirmed only for the test strains belonging to *Alicyclobacillus acidoterrestris.* Nevertheless, both primer sets confirmed no amplified product when using DNA extract derived from 16S rDNA of related strains not belonging to *Alicyclobacillus.*

### (Example 2) Identification of species separated from fruit juice

Fruit juices such as orange, apple, pineapple and plum (Japanese apricot) were added to YSG agar medium (yeast extract: 0.2%, glucose: 0.1%, soluble starch: 0.2%, agar: 1.5%, pH 4.0) and incubation was performed at 45°C for 2-5 days. The bacterial strains were separated from the media and the separated strains were subj ected to nucleic acid amplification and amplified product detection by the LAMP method in the same manner as Example 1. The amplification results are shown in Table 2.

### (Identification by gene analysis)

The separated strains were identified by 16S rDNA gene analysis. After preparing genomic DNA by the method described in Example 1, the separated strains were identified using a MicroSeq 500 16S rDNA kit (product of Applied Biosystems).

**[Table 2]**

| Strain | Al primers | Alat primers | Result |
|---|---|---|---|
| No. 1 | + | + | *A. acidoterrestris* |
| No. 2 | + | + | *A. acidoterrestris* |
| No. 3 | + | - | *A. acidocaldarius* |
| No. 4 | + | - | *A. acidocaldarius* |
| No. 5 | + | + | *A. acidoterrestris* |
| No. 6 | + | - | *A. acidocaldarius* |
| No. 7 | + | - | *A. acidocaldarius* |
| No. 8 | + | - | *A. acidocaldarius* |
| No. 9 | + | - | *A. acidocaldarius* |

| | | | |
|---|---|---|---|
| +: amplified -: not amplified | | | |

As shown in Table 2, the results of gene analysis indicated that all of the separated strains belonged to the *Alicyclobacillus* spp. The results of identification by gene analysis coincided with the results of identification using the Al primers, while amplification with the Alat primers was observed only in strains No.1, No.2 and No.5 which were identified as *Alicyclobacillus acidoterrestris* strains.

### (Example 3) Detection of Alicyclobacillus spp. from fruit juice beverage

After mixing 30 ml of YSG liquid medium with 1 ml (10 cfu/ml) of commercially available apple juice concentrate containing added *Alicyclobacillus acidoterrestris* DSM2498, and stationary culturing was conducted at 45°C for 4-24 hours (enrichment culture).

After 1.0 ml of YSG medium cultured for different culturing times was centrifuged at 12,000 rpm for 5 minutes, the cells were collected. The cells were suspended in 200 µl of TE buffer (10 mM Tris-HCl, 0.1 mM EDTA, pH 8.0), and then centrifugal separation was performed and the cells were rinsed. The rinsed cells were suspended in 100 µl of PrepMan™ Ultra and heat treated at 99°C for 15 minutes, after which they were centrifuged at 12,000 rpm for 5 minutes and the supernatant was used as the DNA sample solution for LAMP method.

One µl portion of the DNA sample solution prepared according to the method described above was subjected to nucleic acid amplification and amplified product detection by the LAMP method using Alat primers, in the same manner as Example 1.

On the other hand, a portion of culture solution was transferred to YSG agar medium for different culturing times, and the viable cells were counted. The amplification results and the viable cell counts are shown in Table 3.

**[Table 3]**

| | Enrichment culture time (hr) | | | | |
|---|---|---|---|---|---|
| | 0 | 4 | 8 | 16 | 24 |
| Detection with Alat | - | - | - | + | + |
| Viable cells (cfu/ml) | 0.3 | <10 | 40 | 1 × 10⁴ | 6 × 10⁵ |
| Viable cell count: Measurement after 3 days of culturing (45°C) in YSG agar medium (cfu/ml) | | | | | |

As shown in Table 3, mere overnight enrichment culture allowed *Alicyclobacillus acidoterrestris* at an initial cell count of no more than 1 cfu/ml of (in YSG liquid medium) to be detected by the LAMP method using the Alat primers. Thus, by detecting a target gene by the LAMP method after short-term enrichment culture (16 hours in the examples), it is possible to selectively detect viable cells without detecting the genes of dead cells.

According to "Thermo Acidophilic Bacilli Standardized Examination Method" (Kaju Kyokaiho, Mar. No. 535, 4-12, 2003), 3-5 days are required for enrichment culture, and 3-5 days are required for discernment of the presence of *Alicyclobacillus acidoterrestris.* Therefore, by employing the detection method of the invention it is possible to drastically shorten the time required for detection and identification of bacteria.

### (Example 4) Identification of appearing bacteria using Rapid Microbe Detection System (RMDS-SPS)

RMDS-SPS (trademark of Millipore-Sapporo Breweries) is an apparatus for rapid detection of microbes utilizing intracellular ATP. We examined the possibility of identifying whether colonies (ultramicro colonies invisible to the naked eye) detected by using this system are *Alicyclobacillus* spp..

A membrane filter was used for filtration of 1 ml of a commercially available apple juice concentrate beverage containing added *Alicyclobacillus acidoterrestris* DSM2498 (10 cfu/ml). The bacteria-laden membrane filter was placed in YSG agar medium and cultured at 45°C for 5-8 hours (enrichment culture). After culturing, the filter section containing bacteria determined using the RMDS-SPS system was cut out and then 2 µl of PrepManTM Ultra was placed on the filter prior to heat treatment at 99°C for 15 minutes, for DNA extraction. The filter was subjected to nucleic acid amplification and amplified product detection by LAMP method using the Alat primers, in the same manner as Example 1. The results are shown in Table 4.

**[Table 4]**

| | Culturing time (hr) | | |
|---|---|---|---|
| | 0 | | 8 |
| Identification | ND | D | D |

| | | | |
|---|---|---|---|
| D: Discernable as *A. acidoterrestris* | | | |
| ND: Not discernable as *A. acidoterrestris* | | | |

As shown in Table 4, using a Rapid Microbe Detection System (RMDS-SPS) for enrichment culture within several hours permitted *Alicyclobacillus acidoterrestris* to be identified by the LAMP method using the Alat primers.

### Industrial Applicability

The present invention allows selective, rapid and convenient identification and detection of *Alicyclobacillus* spp. (especially *Alicyclobacillus acidoterrestris)* which are causative bacteria of spoilage of fruit juice beverages and the like.

Thus, the primer sets and detection method for *Alicyclobacillus* spp. employing them of the invention are useful for product quality control during the production of foods, fruit juice-containing beverages and the like.

## Claims

1. A primer which amplifies the base sequence of a target region selected from any portion of the nucleic acid sequence from base 81 to base 934 of the 16S rDNA of an *Alicyclobacillus* spp., or its complementary strand, the primer being **characterized by** comprising:
(a) a base sequence which functions as a primer by annealing to 16S rDNA of the *Alicyclobacillus* spp., as the first segment, and
(b) a base sequence located at the 5' end of the first segment which is complementary to the nucleic acid sequence at the 3' end of the first segment, as the second segment.

2. A primer set for detection of an *Alicyclobacillus* spp., which comprises an oligonucleotide set consisting of the nucleic acid sequences set forth in SEQ ID NOS: 1 to 4 and is capable of amplifying a specific region of 16S rDNA of the *Alicyclobacillus* spp..

3. A primer set for detection of *Alicyclobacillus acidoterrestris,* which comprises an oligonucleotide set consisting of the nucleic acid sequences set forth in SEQ ID NOS: 5 to 8 and is capable of amplifying a specific region of 16S rDNA of *Alicyclobacillus acidoterrestris.*

4. A primer set for detection of *Alicyclobacillus acidoterrestris,* which comprises an oligonucleotide set consisting of the nucleic acid sequences set forth in SEQ ID NOS: 9 to 13 and is capable of amplifying a specific region of 16S rDNA of *Alicyclobacillus acidoterrestris.*

5. A method for detection of an *Alicyclobacillus* spp. present in a specimen, **characterized by** using a specific region of 16S rDNA of the *Alicyclobacillus* spp. as a target, selectively amplifying the specific region of 16S rDNA by the LAMP method using a primer according to claim 1, and confirming the presence or absence of the amplified product.

6. A method for detection of an *Alicyclobacillus* spp. present in a specimen, **characterized by** using a specific region of 16S rDNA of the *Alicyclobacillus* spp. as a target, selectively amplifying the specific region of 16S rDNA by the LAMP method using a primer set according to claim 2, and confirming the presence or absence of the amplified product.

7. A method for detection of *Alicyclobacillus acidoterrestris* present in a specimen, **characterized by** using a specific region of 16S rDNA of *Alicyclobacillus acidoterrestris* as a target, selectively amplifying the specific region of 16S rDNA by the LAMP method using a primer set according to claim 3, and confirming the presence or absence of the amplified product.

8. A method for detection of *Alicyclobacillus acidoterrestris* present in a specimen, **characterized by** using a specific region of 16S rDNA of *Alicyclobacillus acidoterrestris* as a target, selectively amplifying the specific region of 16S rDNA by the LAMP method using a primer set according to claim 4, and confirming the presence or absence of the amplified product.

9. A method for identification of an *Alicyclobacillus* spp., **characterized by** carrying out enrichment cultivation of a specimen, separating a DNA sample from the appearing bacteria, subjecting the DNA sample to amplification reaction by the LAMP method using a primer according to claim 1, amplifying the specific region of 16S rDNA of the *Alicyclobacillus* spp., and confirming the presence or absence of the amplified product.

10. A method for identification of an *Alicyclobacillus* spp., **characterized by** carrying out enrichment cultivation of a specimen, separating a DNA sample from the appearing bacteria, subjecting the DNA sample to amplification reaction by the LAMP method using a primer set according to claim 2, amplifying the specific region of 16S rDNA of the *Alicyclobacillus* spp., and confirming the presence or absence of the amplified product.

11. A method for identification of *Alicyclobacillus acidoterrestris,* **characterized by** carrying out enrichment cultivation of a specimen, separating a DNA sample from the appearing bacteria, subjecting the DNA sample to amplification reaction by the LAMP method using a primer set according to claim 3, amplifying the specific region of 16S rDNA of *Alicyclobacillus acidoterrestris,* and confirming the presence or absence of the amplified product.

12. A method for identification of *Alicyclobacillus acidoterrestris,* **characterized by** carrying out enrichment cultivation of a specimen, separating a DNA sample from the appearing bacteria, subjecting the DNA sample to amplification reaction by the LAMP method using a primer set according to claim 4, amplifying the specific region of 16S rDNA of *Alicyclobacillus acidoterrestris,* and confirming the presence or absence of the amplified product.

13. A kit for detection of an *Alicyclobacillus* spp. or *Alicyclobacillus acidoterrestris,* **characterized by** comprising at least a primer or a primer set according to any one of claims 1 to 4, strand-displacing DNA polymerase, dNTPs and reaction buffer.


SEQUENCE LISTING

<110>  Sapporo Breweries Ltd.
       Eiken Chemical Co. Ltd.

<120>  Primers for detection of Alicyclobacillus strain

<130>  FP04-0384

<150>  JP 2003-349772
<151>  2003-10-08

<160>  14

<170>  PatentIn version 3.1

<210>  1
<211>  40
<212>  DNA
<213>  Artificial sequence

<220>
<223>  primer

<400>  1
ttgggtttcc ttcggcactg agataccctg gtagtccacg                          40


<210>  2
<211>  39
<212>  DNA
<213>  Artificial sequence

<220>
<223>  primer

<400>  2
ataagcactc cgcctgggga gcccccgtca attcctttg                           39


<210>  3
<211>  19
<212>  DNA
<213>  Artificial sequence

<220>
<223>  primer

<400>  3
gtggggagca aacaggatt                                                 19


<210>  4
<211>  19
<212>  DNA
<213>  Artificial sequence

<220>
<223>  primer

<400>  4
acatgctcca ctgcttgtg                                                 19


<210>  5
<211>  40
<212>  DNA
<213>  Artificial sequence

<220>
<223>  primer

<400>  5
acaaggagct ttccactctc caagaaggcc ttcgggttgt                          40


<210>  6
<211>  40

<212>  DNA
<213>  Artificial sequence

<220>
<223>  primer

<400>  6
tgagacggta ccgagtgagg acgcttgccc cctacgtatt                              40


<210>  7
<211>  17
<212>  DNA
<213>  Artificial sequence

<220>
<223>  primer

<400>  7
caagcctgac ggagcaa                                                       17


<210>  8
<211>  18
<212>  DNA
<213>  Artificial sequence

<220>
<223>  primer

<400>  8
cccagtgatt ccggacaa                                                      18


<210>  9
<211>  43
<212>  DNA
<213>  Artificial sequence

<220>
<223>  primer

<400>  9
aacacaagta gatgcctacc cgcaatctgc ctttcagact gga                          43


<210>  10
<211>  43
<212>  DNA
<213>  Artificial sequence

<220>
<223>  primer

<400>  10
ttgaaagatg caactgcatc gctccgttac ctcaccaact agc                          43


<210>  11
<211>  19
<212>  DNA
<213>  Artificial sequence

<220>
<223>  primer

<400>  11
cggacgggtg agtaacacg                                                     19


<210>  12
<211>  18
<212>  DNA
<213>  Artificial sequence

<220>
<223>  primer

<400> 12
tacgcatcgt cgccttgg 18

<210> 13
<211> 20
<212> DNA
<213> Artificial sequence

<220>
<223> Primer

<400> 13
attagcaccc gtttccgagt 20

<210> 14
<211> 1514
<212> DNA
<213> alicyclobacillus

<400> 14
agagtttgat cctggctcag gacgaacgct ggcggcgtgc ctaatacatg caagtcgagc 60
gagcccttcg gggctagcgg cggacgggtg agtaacacgt gggcaatctg cctttcagac 120
tggaataaca ctcggaaacg ggtgctaatg ccggataata cacgggtagg catctacttg 180
tgttgaaaga tgcaactgca tcgctgagag aggagcccgc ggcgcattag ctagttggtg 240
aggtaacggc tcaccaaggc gacgatgcgt agccgacctg agagggtgac cggccacact 300
gggactgaga cacggcccag actcctacgg gaggcagcag tagggaatct tccgcaatgg 360
gcgcaagcct gacggagcaa cgccgcgtga gcgaagaagg ccttcgggtt gtaaagctct 420
gttgctcggg gagagcgaca aggagagtgg aaagctcctt gtgagacggt accgagtgag 480
gaagccccgg ctaactacgt gccagcagcc gcggtaatac gtagggggca agcgttgtcc 540
ggaatcactg ggcgtaaagc gtgcgtaggc ggttgtgtaa gtctgaagtg aaagtccaag 600
gctcaacctt gggattgctt tggaaactgc atgacttgag tgctggagag gcaaggggaa 660
ttccacgtgt agcggtgaaa tgcgtagata tgtggaggaa taccagtggc gaaggcgcct 720
tgctggacag tgactgacgc tgaggcacga aagcgtgggg agcaaacagg attagatacc 780
ctggtagtcc acgccgtaaa cgatgagtgc taggtgttgg ggggacacac cccagtgccg 840
aaggaaaccc aataagcact ccgcctgggg agtacggtcg caagactgaa actcaaagga 900
attgacgggg gcccgcacaa gcagtggagc atgtggttta attcgaagca acgcgaagaa 960
ccttaccagg gcttgacatc cctctgaccg gtgcagagat gtaccttccc ttcggggcag 1020
aggagacagg tggtgcatgg ttgtcgtcag ctcgtgtcgt gagatgttgg gttaagtccc 1080
gcaacgagcg caacccttga tctgtgttac cagcacgtag aggtggggac tcacaggtga 1140
ctgccggcgt aagtcggagg aaggcgggga tgacgtcaaa tcatcatgcc ctttatgtcc 1200
tgggctacac acgtgctaca atgggcggta caacgggaag cgaagccgcg aggtggagca 1260
aaacctaaaa agccgttcgt agttcggatt gcaggctgca actcgcctgc atgaagccgg 1320
aattgctagt aatcgcggat cagcatgccg cggtgaatcc gttcccgggc cttgtacaca 1380
ccgcccgtca caccacgaga gtcggcaaca cccgaagtcg gtgaggtaac cgttatggag 1440
ccagccgccg aaggtggggt tgatgattgg ggtgaagtcg taacaaggta gccgtatcgg 1500
aaggtgcggt tgga 1514